# EUROPEAN PATENT SPECIFICATION

(11) **EP 3 760 238 B1**
(45) Date of publication and mention of the grant of the patent: **20.11.2024**
(21) Application number: 20183536.0
(22) Date of filing: 01.07.2020
(51) Int. Cl.: A41D 31/12, A61F 13/15, A61L 15/22, A47G 9/02, A41B 17/00, A61L 15/42

(54) **A DEVICE FOR MOISTURE REGULATION ON A PORTION OF SKIN OF A LIVING BEING, AS WELL AS AN ARTICLE AND SET COMPRISING SUCH A DEVICE**
VORRICHTUNG ZUR FEUCHTIGKEITSREGULIERUNG AUF EINEM TEIL DER HAUT EINES LEBEWESENS SOWIE ARTIKEL UND SET MIT SOLCH EINER VORRICHTUNG
DISPOSITIF DE RÉGULATION DE L'HUMIDITÉ SUR UNE PARTIE DE LA PEAU D'UN ÊTRE VIVANT, AINSI QU'UN ARTICLE ET UN ENSEMBLE COMPRENANT UN TEL DISPOSITIF

(30) Priority: 02.07.2019 NL 2023421
(43) Date of publication of application: 06.01.2021
(73) Proprietor: BelSmit B.V., 6438 AE Oirsbeek (NL)
(72) Inventor: Smit, Leendert, 6438 AE Oirsbeek (NL)
(74) Representative: Vandevijver, Pascale

(56) References cited:
- GB-A- 2 473 321
- US-A1- 2016 333 505
- US-B2- 8 646 459

## Description

### FIELD OF THE INVENTION

The invention relates to a device for moisture regulation on a portion of skin of a living being, which device is at least to be located between the portion of skin of the living being and an object proximate that portion of the skin of the living being, the device comprising at least a first layer comprising of a tricot fabric and directed in use with an outer side towards the portion of the skin and at least a second layer being in contact with an inner side of the first layer at a side remote of the portion of the skin.

The invention also relates to an article and set comprising such a device.

### BACKGROUND OF THE INVENTION

By a device and article known from US8646459, comprises two layers of tricot fabric, each layer having a shiny side with a close-knit design with ribs running in the cross direction while employing an inter-loop yam pattern. The shiny sides of the two layers of fabric face each other. Each layer of fabric has been made in a machine direction. The two layers of fabric are positioned such that the machine direction of one layer is perpendicular to the machine direction of the other layer.

One suitable type of fabric is a three-bar tricot fabric consisting of 85% 40-denier semi dull nylon and 15% 140- 15 denier spandex.

This known device has the object to reduce the likelihood of ulcer formation on a portion of skin of a living being by reducing the friction between the portion of skin of the living being and an object proximate that portion of the skin of the living being.

Although friction plays an important role in reducing the likelihood of ulcer formation, other features seems to be more relevant to prevent ulcer formation.

### SUMMARY OF THE INVENTION

At least one of the objects of the invention is to provide a device for moisture regulation on a portion of skin of a living being.

This object is accomplished with the device according to the invention in that
- the first layer directed in use towards the portion of the skin
   o has a moisture regain of less than 5%, more preferably less than 3,5% even more preferably less than 1,5%,
   o has wicking according to DIN53924, with a relation between a vertical rising height H (cm) and the square root of a rising time T (sec) being H = K₁ *T^{0,5,} wherein 0,09 ≤ K₁ ≤ 0,2,

- the second layer being in contact with the first layer at a side remote of the portion of the skin
   o comprises a spacer fabric being porous in a direction perpendicular as well as parallel to the surface of the second layer,
   o has a compression resistance of ≥4 kPa, more preferably ≥10.5 kPa at 40% compression,
   o has wicking according to DIN53924, with a relation between a vertical rising height H (cm) and the square root of a rising time T (sec) being H = K₂*T^{0,5} wherein K₂ > K₁ , preferably K₂ ≥ 0,2, more preferably K₂ ≥ 0,35, even more preferably K₂ ≥ 0,49.

The current invention uses a combination of both a knitted tricot fabric with a spacer fabric, whereby the individual fabrics as well as their positioning is optimized such that the interaction between the two fabrics enables both an optimized moisture transfer as well as a good sliding effect in order to maintain a low shear on the skin.

The first layer directed in use towards the portion of the skin has as main function to remove as soon as possible moisture away from the skin of user towards the second layer. The moisture is water or sweat from the user.

It has found out that the wicking of the first layer must be within a specific range. If a tricot with a very high wicking rate is chosen, then it appears that the drying time of the combined structure starts to increase rapidly (drying rate reduces). It appears that first layers with an inner tricot fabrics with very high wicking rate tend to hold on to the moisture and prevent it from leaving the fabric. So the wicking rate should be limited.

The most optimal tricot of the first layer is therefore a tricot with a surface structure that easily allows moisture to pass through, yet is not "superabsorbent" (has a minimum level of moisture uptake by itself) in order to prevent that it will tend to hold on to the absorbed moisture. The fabric of the first layer in this way enables maximum transfer of moisture directly towards the spacer fabric, away from the skin.

By preserving a lower wicking rate for the first layer compared to the second layer AND ensuring a good exchange contact between first and second layer, the moisture from the skin is actually pulled/sucked through the first layer, therefore keeping the skin-surface dry and cool.

The use of two layers in which the first layer on the skin functions as a dry barrier is a surprising effect of the present invention.

The second layer has as main function to remove as soon as possible the moisture from the first layer in a direction parallel to the second layer. Therefore the second layer has a higher wicking rate than the first layer. Preferably K₂ ≥ 0,2, more preferably K₂ ≥ 0,35, even more preferably K₂ ≥ 0,49.

Due to the porosity of the second layer it has an open structure guaranteeing open channels in the second layer for water and vapour in the direction into and along the second layer.

By doing so the micro-climate on the skin is being optimized. By removing the moisture, the temperature and humidity of the skin is being lowered. This is important as it has been demonstrated that the micro-climate greatly affects the risk of pressure ulcer formation as well as the level of comfort. It has turned out that the device according to the invention provides both cooling and ventilation of the skin.

The moisture regain of less than 5%, more preferably less than 3,5% even more preferably less than 1,5%, of the first layer has the advantage that nearly no moisture is being absorbed by the first layer but will be guided to the second layer and be spread in the second layer.

The first layer has a wicking according to DIN53924, with a relation between a vertical rising height H (cm) and the square root of a rising time T (sec) being H = K₁*T^{0,5} wherein 0,09 ≤ K₁ ≤ 0,2.

With such a wicking value moisture like water and sweat will be easily spread throughout the first layer based on capillary function of the first layer. By spreading the moisture over the first layer, the local humidity on the skin will be reduced. Furthermore, by spreading the moisture over the first layer, the amount of moisture that needs to be transported from the first layer to the second layer per square centimer is reduced.

With a compression resistance of ≥4 kPa, more preferably ≥10.5 kPa at 40% compression, the second layer will under normal pressures from the weight of a user on the device, maintain an open enough structure to be able to transport moisture through capillary movement in the direction along the second layer. For obese users the compression resistance must be higher than for a person having an average weight. The compression of the spacer fabric will result in some spreading of the local pressure, distributing it horizontally, thus reducing the maximum pressure on the skin and thus reducing ulcer formation.

The spacer fabric can for example comprise two adjacent knitted layers interconnected by an open structure, a directly spun filament mat or a resilient non-woven. Such a spacer fabric will result in lowering of the shear pressure. In this manner, the movement of the filament layers with respect to each other in a direction parallel to the surface of the filament layers will result in some level of shear reduction thus reducing ulcer formation.

Due to the moisture regulation by the device according to the invention, the device is suitable to reduce the likelihood of ulcer formation. Furthermore the device according to the invention is suitable to remove sweat from the skin, for example by night sweats.

An embodiment of the device according to the invention is characterized in that the inner side of the first layer is smoother and/or denser than the outer side of the first layer.

The smoothness has influence on the friction between the inner side and the second layer and between the outer side and the skin. Furthermore, the smoother surface will provide for a larger effective contact area with the underlying second layer, thus also increasing the transfer of moisture towards this second layer. This provides for a dry skin-feel.

Another embodiment of the device according to the invention is characterized in that the tricot of the first layer has a warp-knitted structure, preferably a warp-knitted two-way structure.

It was surprisingly found out that the use of a warp-knitted structure, preferably a warp-knitted two-way structure performs much better on the skin than the more common weft knitted tricot fabrics as used in sports clothes. A warp knitted plain tricot structure is knitted with two full sets of knitting needles or guide bars. Depending on the configuration of laps different two bar structures are possible, for instance two bar tricot (also known as half jersey), Satin, Sharkskin, Reverse Locknit and Locknit (also known as two way or Charmeuse). Within these possible structures, a warp knitted, two-way structure called Locknit or Charmeuse is the preferred structure.

Two-way tricot structures are known under various names, most prominently "Locknit" in the US and "Charmeuse" in Europe. These fabrics are made on so called two-bar warp knitting machines, which enable the construction of a fabric with features a different structure on the outer side versus the inner side.

The inner side is visually similar to a regular knitted structure as it is obtained in general, cheaper weft knitted fabrics. The inner side is often called front side in the knitting industry. In the same manner the outer side is often called backside in the knitting industry. In the present invention the so called backside is surprisingly used on the outside so on the front of the device according to the invention. The outside features a structure with the visual appearance of twisted rope structures, running in the machine direction. The rope structures can be placed tightly next to each other or with a small distance in-between - depending on the actual knitting parameters (number of stitches per inch in machine direction and cross direction) and yarn construction (thickness in denier, number of filaments).

The first layer of such a warp-knitted tricot structure can be either with or without an inner mesh from a more elastic fiber (the elastic inner mesh can provide for additional elasticity which is very important for applications in which the embodiment of the fabric technology is meant to shape itself closely around the body without forming wrinkles).

Another embodiment of the device according to the invention is characterized in that between the inner side and outer side of the first layer an elastic mesh is located.

Such mesh can provide for additional elasticity which is very important for applications in which the device according to the invention is meant to shape itself closely around the body without forming wrinkles).

Another embodiment of the device according to the invention is characterized in that the mesh is made of a thermoplastic co-polyester elastomer (TPE-C).

This will give the device an good washability and recyclability.

Another embodiment of the device according to the invention is characterized in that the tricot of the first layer is made of a mixture of polyethylene terephthalate (PET) fibers and polybutylene terephthalate (PBT) fibers.

This will give a fabric with good washability, which enables commercial use of the device in both consumer as well as healthcare environment. The amount of polyethylene terephthalate (PET) fibers can be between 30-70% and the amount of polybutylene terephthalate (PBT) fibers can be between 70-30%. It also is recyclable.

Another embodiment of the device according to the invention is characterized in that yarns of the tricot of the first layer are preferably less than 120 denier, more preferably less or equal than 50 denier.

The first layer of the two way structure is preferably made with fine yarns of a limited denier, which will ensure the construction of tightly knitted cables on the outer side, where capillary absorption within the cable structure will be prevented and sweat can only be transported through the voids in between the cables. The inner side of the fabric, due to its knitting structure, will have more capillary spaces in between the individual yarns, which will pull the liquid from in between the outer side cables.

If the denier is higher that 120, the yarns are thicker, a much more open structure is obtained, not only in between the yarns but also within the yarns. This will result in the very high wieking fabrics that keep the moisture enclosed and therefore do not dry as well. Such fabrics constructions with thicker yarns thus lead to fabrics with higher moisture regain and are therefore not suitable for pressure ulcer prevention and comfort enhancement during sweating.

Preferably the first layer directed in use towards the portion of the skin has a contact angle of less than 90 degrees with water.

The contact angle of less than 90 degrees with water of the first layer has the advantage that water and sweat will easily be spread over the material that is used for the manufacture of the first layer.

Preferably the first layer has a moisture regain of less than 1,5% weight increase.

This further reduces the chance that moisture will remain in the first layer.

Preferably the first layer has an air permeability of at least 50 liter*metre⁻²*seconds⁻¹, preferably of at least 375 liter*metre⁻²*seconds⁻¹.

The air permeability has a large influence on the possibility of the first layer to move moisture through the first layer towards the second layer. It has found out that with the indicated air permeability the moisture will be moved relatively quickly through the first layer.

Preferably the first layer has a water vapour permeability of at least 17 mg*centimetre⁻²* hour⁻¹, preferably of at least 25 mg*centimetre⁻²* hour⁻¹ , more preferably of at least 55 mg*centimetre⁻²* hour⁻¹ .

The water vapour permeability has a large influence on the possibility of the first layer to move moisture through the first layer towards the second layer. It has found out that with the indicated water vapour permeability the moisture will be moved relatively quickly through the first layer.

Preferably the first layer has a drying speed of ≥ 1 delta%/minute, preferably ≥ 1,5 delta%/minute.

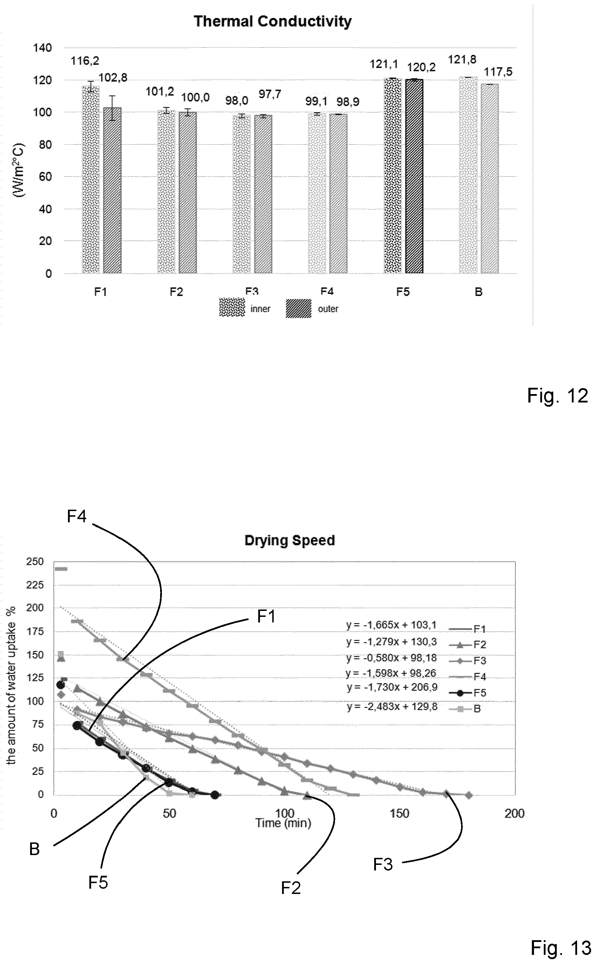

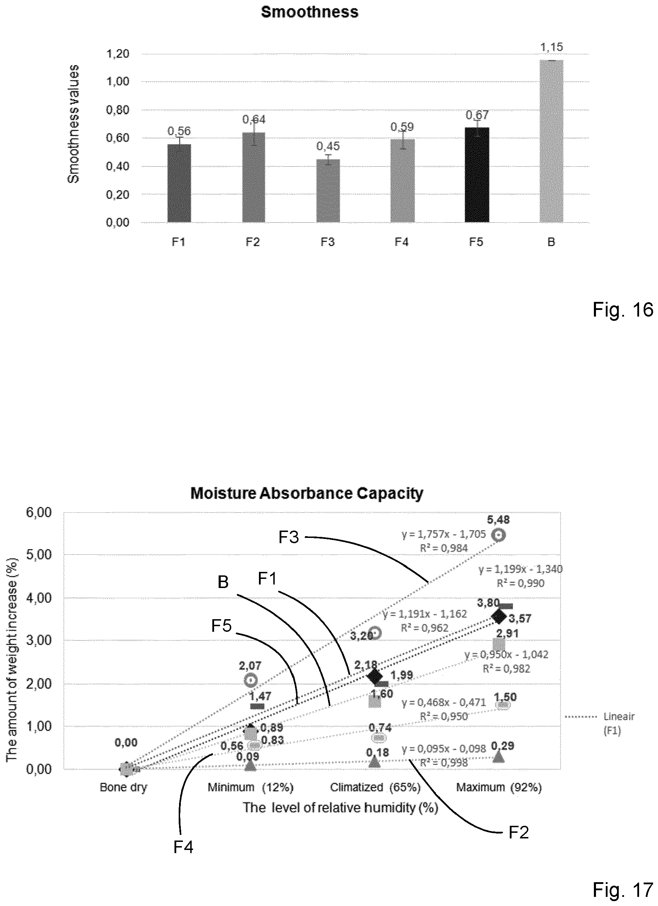

Delta% is defined as the relative surplus amount of water in the fabric after immersion for 3 minutes in liquid (as graphically shown in figure 13 here below) versus its climatized equilibrium water uptake (as measured and shown in figure 17 here below).

By having such a drying speed, the first layer will get dry relatively quickly.

Preferably the first layer has an average surface friction coefficient on a side directed towards the portion of the skin of less than 0,2.

Friction plays an important role in the reduction of ulcer formation. It has found out that especially the average surface friction coefficient on a side of the first layer directed towards the portion of the skin should be as low as possible.

Preferably the first layer has a smoothness of less than 1, preferably less than 0,7.

Such a relatively low smoothness will lead to a reduced average surface friction coefficient. In addition a low smoothness provides for a comfortable feel of the clothing, easiness putting it on the body. It also eases sliding of the layers over each other, which reduces shear on the skin.

Preferably the first layer has a thermal conductivity of ≥ 95 W·m⁻² · °C⁻¹, preferably of ≥ 120 W·m⁻² · °C⁻¹.

With such a thermal conductivity heat from the skin of the user will easily be transported from the skin to the first layer and through the first layer to the second layer. Reduction of the temperature of the skin reduces the likelihood of ulcer formation as well as in improving comfort during extensive night sweating.

Another embodiment of the device according to the invention is characterized in that the first layer and/or the second layer comprises a synthetic polymer knitting of nylon, polyester, polypropylene, polyethylene, or a combination thereof with an elastic fiber like spandex, elastane, lycra, a thermoplastic elastomer etc.

It has been found out that a device made of these materials fulfils the requirements as set out above to reduces the likelihood of ulcer formation.

Another embodiment of the device according to the invention is characterized in that the first layer and/or the second layer comprises a content of elastic fiber between 2 - 30 %.

With such amount of elastic fiber the elasticity of the layers is such that the layers can easily be the stretched, so that the layers can easily comply to the shape of the skin without folds. Such folds would increase the likelihood of ulcer formation.

Another embodiment of the device according to the invention is characterized in that the first layer and/or the second layer comprises 82-100 % polyester and 0-18 % elastic fiber.

It has been found out that such combination provides very good results in reducing the likelihood of ulcer formation.

Another embodiment of the device according to the invention is characterized in that the first layer and/or the second layer comprises a 30-70 % polyethylene, 30-70 % polyester and 0-20 % elastic fiber.

The second layer has as main object to form a ventilation channel to remove moisture and heat from the first layer in a direction parallel to the first and second layer and towards a location on the second layer where the moisture and heat can be delivered to the environment around the user.

Preferably the first layer and/or the second layer has an aerial weight of less than 250 gram*metre⁻², preferable less than 200 gram*metre⁻², optimally less than 150 gram*metre⁻².

Such layers are relatively light and suitable to be worn as clothing, for example.

Another embodiment of the device according to the invention is characterized in that the second layer has a stretchability similar to the stretchability of the first layer.

By having similar stretchabilities, the risk of folds in one of the two layers when stretching or bending the layers is limited.

Preferably the second layer has a weight of less than 600 gram*metre⁻², preferable less than 500 gram*metre⁻².

Preferably the weight of each layer is as low as possible. This increases the comfort as well as the visual appearance of the clothing made of the device according to the present invention.

Another embodiment of the device according to the invention is characterized in that the second layer has a thickness between 2,5 and 4 millimetre, preferable between 2,5 and 3,5 millimetre.

With such thickness, the second layer will be thin enough to be suitable for clothing and thick enough to have the desired features to remove heat and moisture from the first layer to the environment.

It is also possible that the first and second layer are integrated into one layer.

The first and second layers can be made as separate layers which are connected to each other. However it is also possible to made the device out of one layer whereby the side directed to the skin of the user forms the first layer providing the technical features as described above of said first layer, whilst the side directed away from the skin of the user forms the second layer providing the technical features as described above of said second layer.

The invention also relates to an article comprising a device according to the invention, like
- articles of clothing,
- articles of covering cushioning devices like sheets, mattresses and pillows,
- articles of covering the skin touching surfaces of various medical devices, like orthotics, braces, etc.

All such kind of articles are intended to be located between the skin of a user and the environment. At locations were contact and pressure between the skin of a user and the environment ulcer formation might occur, the article comprising the device according to the invention will prevent such ulcer formation. Furthermore such articles are also suitable to remove sweat from the skin, for example by night sweats by persons not having a risk to ulcer formation.

The invention also relates to a set comprising a device according to the invention well as a clothing of a similar material as the first layer.

In such embodiment the clothing can be made of the same or similar material as the first layer. This will render the same highly effective moisture transporting effect.

### BRIEF DESCRIPTION OF THE DRAWINGS

The device and article according to the invention will further be explained with reference to the drawings, wherein,
figure 1 is a perspective view of a first embodiment of a device according to the invention,
figure 2 is a perspective view of the first embodiment of a device according to the invention, as shown in figure 1, positioned near a skin of a user,
figure 3 is a perspective view of a third embodiment of a device according to the invention,
figures 4A and 4B are a rear view and side view of a first embodiment of an article according to the invention comprising the device as shown in figure 1, as worn by a user,
figure 5 is a side view of a second embodiment of an article according to the invention comprising the device as shown in figure 1, as worn by a user,
figure 6 is a top view of a third embodiment of an article according to the invention comprising the device as shown in figure 1,
figure 7 is an overview of several materials of a first layer of the device according to the invention,
figure 8 is a spider diagram showing several features of the materials as shown in figure 7,
figure 9 is a graph regarding wieking of some materials as shown in figure 7,
figure 10 is a graph regarding air permeability of the materials as shown in figure 7,
figure 11 is a graph regarding water vapour permeability of the materials as shown in figure 7,
figure 12 is a graph regarding thermal conductivity of the materials as shown in figure 7,
figure 13 is a graph regarding drying speed of the materials as shown in figure 7,
figure 14 is a graph regarding drying speed versus areal density of the materials as shown in figure 7,
figure 15 is a graph regarding friction of the materials as shown in figure 7,
figure 16 is a graph regarding smoothness of the materials as shown in figure 7,
figure 17 is a graph regarding moisture regain of the materials as shown in figure 7,
figure 18 is an overview of several materials of a second layer of the device according to the invention,
figure 19 is a graph regarding stretchability of the materials as shown in figure 18.
figure 20 is a photo showing the inner side and outer side of the first layer,
figures 21A-21C are schematic views of the production of a two way structure of the first layer,
figure 22 is a side view of another embodiment of an article according to the invention comprising the device as shown in figure 1, as worn by a user.

In the drawings, like reference numerals refer to like elements.

### DESCRIPTION OF PREFERRED EMBODIMENTS

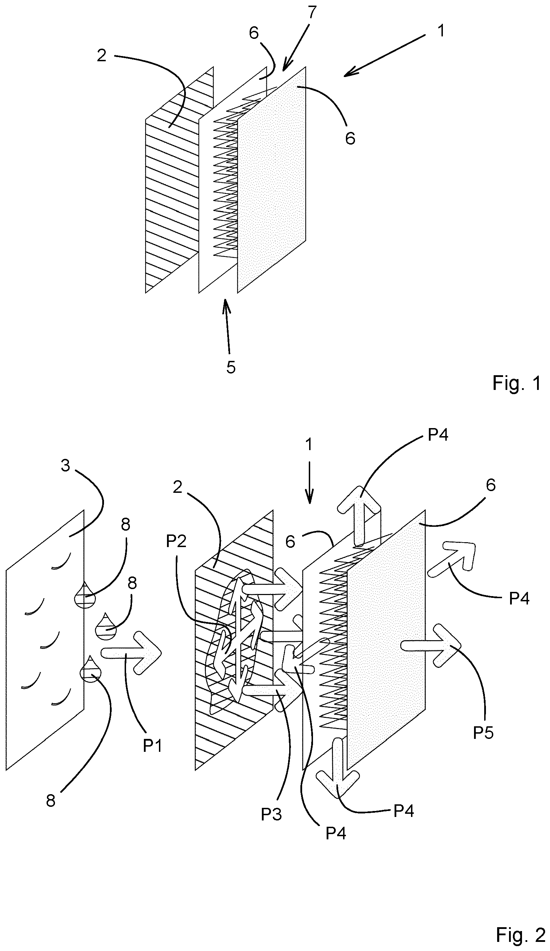

Figures 1 and 2 are perspective views of a first embodiment of a device 1 according to the invention. The device 1 comprises a first layer 2 directed in use towards a portion of a skin 3 of a user 4 and a second layer 5 being in contact with the first layer 2 at a side remote of the portion of the skin 3. The first layer 2 is thinner than the second layer 5.

The second layer 5 is made of a spacer fabric being porous in a direction perpendicular as well as parallel to the surface of the second layer 5. The spacer fabric comprises two adjacent knitted layers 6 interconnected by an open structure 7 for transporting moisture and air.

As can be seen in figure 2, the skin 3 is located opposite and against the first layer 2 of the device 1 according to the invention. Moisture 8 like sweat from the skin 3 is being transported in the direction indicated by arrow P1 towards and onto the first layer 2 of the device 1.

Due to the features of the first layer 2, as will be explained here below, the moisture 7 is spread over the first layer 2 in directions indicated by the arrow P2 being parallel to the surface of the first layer 2 as well as in a direction indicated by the arrow P3 being perpendicular to the surface of the first layer 2 and directed towards and onto the second layer 5 of the device 1.

Due to the features of the second layer 5, as will be explained here below, the moisture 7 is spread inside the second layer 5 in directions indicated by the arrow P4 and opposite thereto being parallel to the surface of the second layer 5 as well as in a direction indicated by the arrow P5 being perpendicular to the surface of the second layer 5 and directed towards the environment.

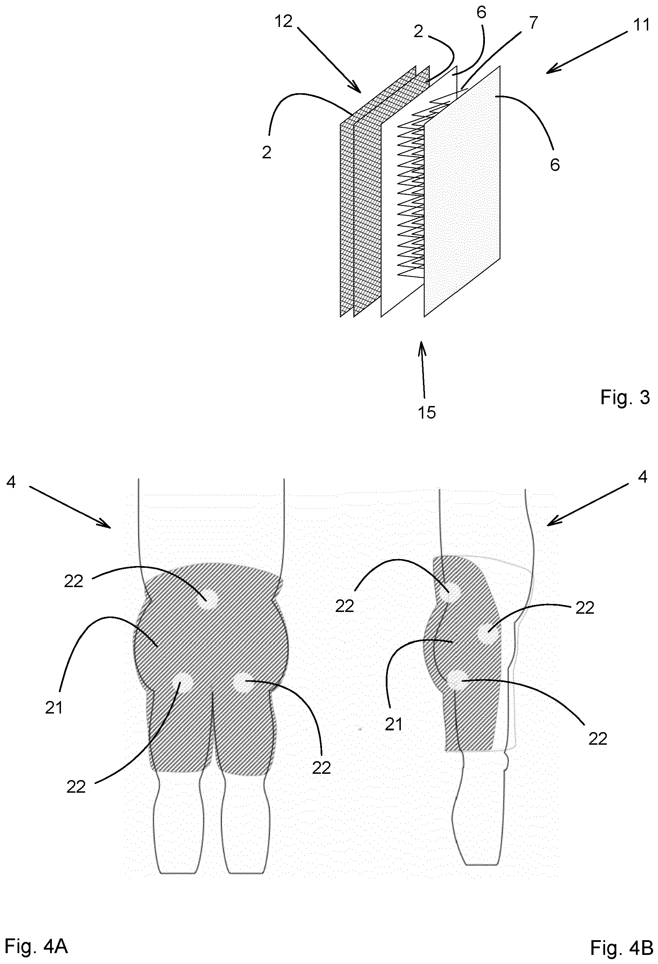

Figure 3 shows a second embodiment of a device 11 according to the invention. The device 11 comprises a first layer 12 directed in use towards a portion of a skin 3 of a user 4 and a second layer 15 being in contact with the first layer 12 at a side remote of the portion of the skin 3. The first layer 12 is thinner than the second layer 15. The first layer 12 comprises a number of layers 2 of the device 1 of the first embodiment of the invention. By the device 11 the second layer 15 is identical to layer 5 of the device 1 of the first embodiment of the invention. However, it is also possible that the second layer 15 comprises a number of layers 5 of the device 1 of the first embodiment of the invention. Multiple first and/or second layers can be used in special circumstances, for example in case where very high point pressures can be envisioned in clothing articles for obese people as well as in the design of foot-soles.

In these cases some second layers 15 can have lower or higher compression resistances - as these will be tuned in the specific design, optimizing the balance between maintaining an open ventilation structure and redistributing the pressure maximum on the skin.

Figures 4A and 4B show a rear view and side view of a first embodiment of an article according to the invention comprising the device as shown in figure 1, as worn by a user 4. The article comprises a pair of trousers 21. On said pair of trousers 21 several spots 22 are indicated where the risk of ulcer formation is relatively large. Especially at those spots, the device 1 according to the invention must have the desired technical features.

Such pair of trousers 21 is suitable for a user lying down in a bed over a long time, for example during illness. Under such circumstances most persons will lie on their back or side. The front side of the pair of trousers 21 does not to be provided with the device 1 according to the invention. If a trouser design is made for a person lying face-down, the trouser design will be made where the device 1 of the invention is used in covering the body parts with highest pressure load in that position.

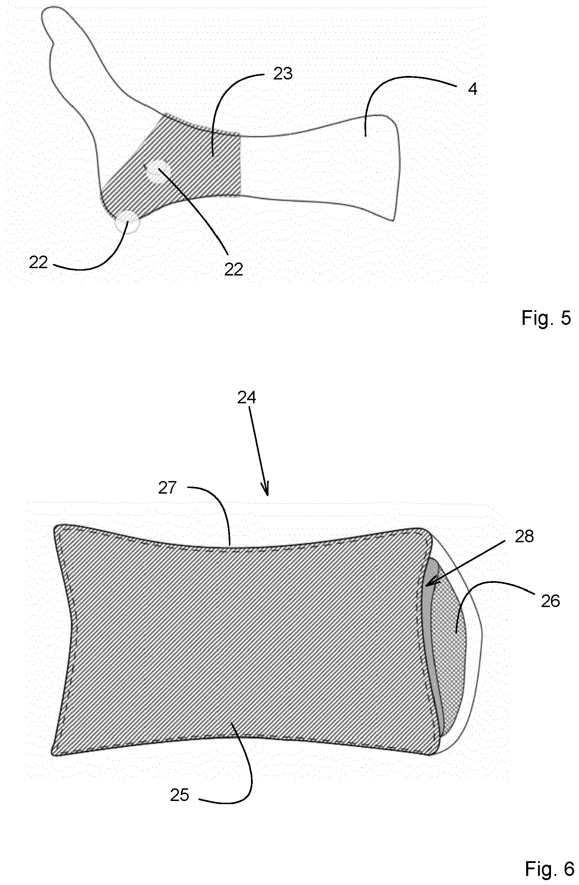

Figure 5 shows a side view of a second embodiment of an article according to the invention comprising the device as shown in figure 1, as worn by a user 4. The article comprises a kind of sock 23 as worn on an ankle of a user 4. On said kind of sock 23 several spots 22 are indicated where the risk of ulcer formation is relatively large. Especially at those spots, the device 1 according to the invention must have the desired technical features.

Figure 5 shows a side view of a second embodiment of an article according to the invention comprising the device as shown in figure 1. The article comprises a pillow case 24.

The pillow case 24 comprises two sheets 25made of the device 1 according to the invention, which sheets 25 are connected to each other by means of a seam 27. The seam 27 extends along two longitudinal sides and one short side of the pillow case 24. On the other short side an opening 28 is present to be able to insert for example a normal and well known pillow 26 in the pillow case 24.

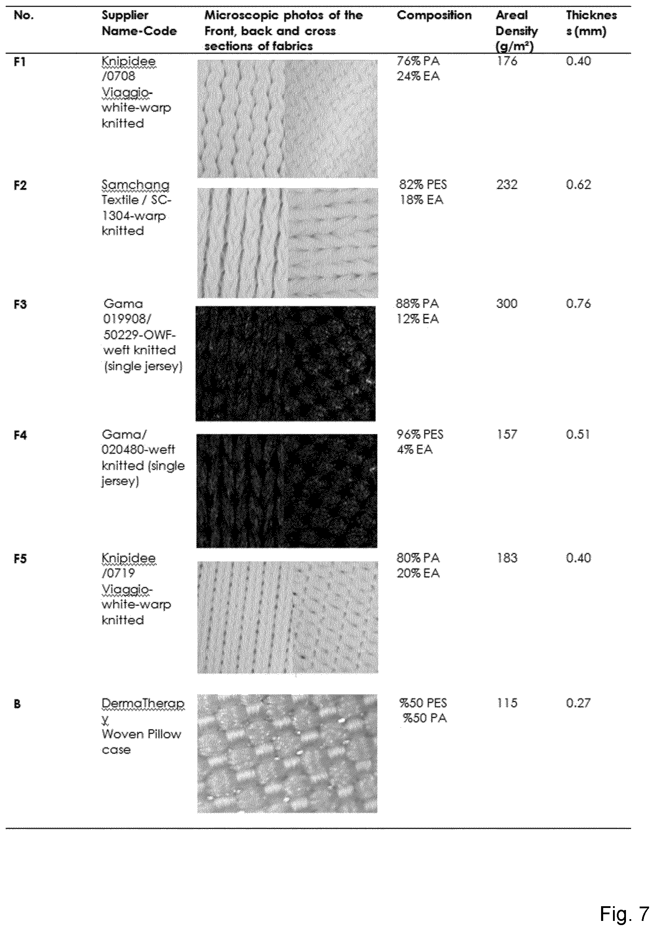

Figure 7 shows an overview of several materials F1-F5, B of the first layer 2 of the device 1 according to the invention.

Material B is used as benchmark. It is a woven pillow made by DermaTherapy. It is made of 50% polyester (PES) and 50% polyamide (PA), with an areal density of 115 gram*metre⁻² and a thickness of 0,27 millimetre. The intention of the present invention is to provide a device 1 with better results than the benchmark material B.

The materials F1-F5 are made of polyester (PES) or polyamide (PA) combined with spandex (EA). Spandex (EA) is also known as elastane or lycra. The composition, areal density and thickness are indicated in figure 7.

These are products from Knipidee International BV (NL), Samchang Textile Co., Ltd. (KR) and of the trademark Gamateks and DermaTherapy respectively.

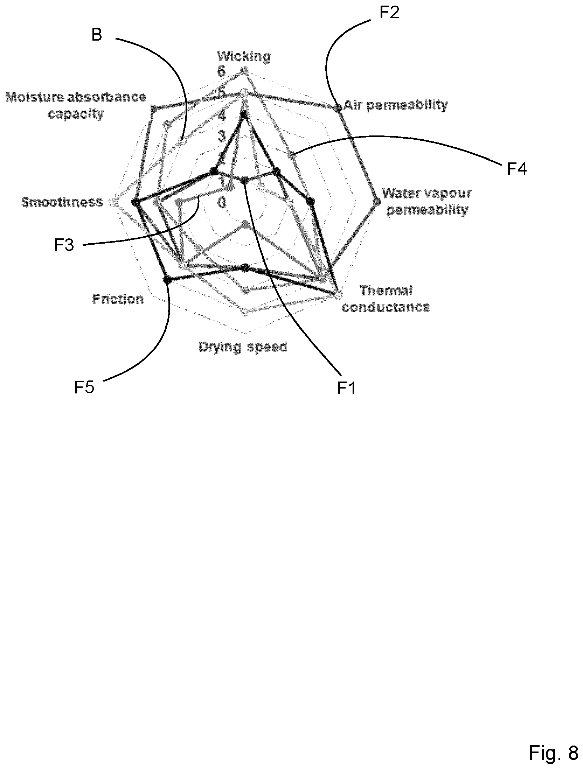

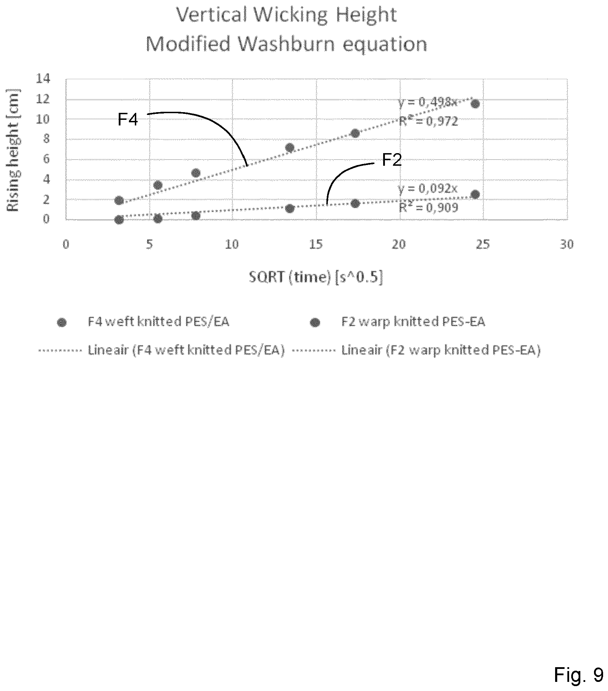

Figure 8 is a spider diagram showing several technical features of the materials as shown in figure 7. For each feature a value between o and 6 is given for each material F1-F5, B based on the measurements and technical information as given in the figures 9-18. The higher the value, the better the respective feature of a material is suitable to prevent the likelihood of ulcer formation. The following technical features are indicated in the spider diagram:
- wicking
- air permeability
- water vapour permeability
- thermal conductance
- drying speed
- friction
- smoothness
- moisture regain

All these technical features are more or less of importance to prevent the likelihood of ulcer formation. Based on the spider diagram, the most preferred material is F2.

The several technical features of the first layer 2 will be explained with reference to the figures 9-18.

Figure 9 shows a graph regarding wicking of materials as shown in figure 7.

The layer 2 according to the invention has a wicking according to DIN53924, with a relation between a vertical rising height H (cm) and the square root of a rising time T (sec) being H = K₁ *T^{0,5} wherein 0,09 ≤ K₁ ≤ 0,2, Wicking provides information about the possibility of the material to spread water over and inside the first layer 2 based on capillary function of the first layer 2.

F2 and F4 are both made of PES/EA. The difference between F2 and F4 is that F2 is warp knitted whilst F4 is weft knitted. F2 is the Charmease fabric as discussed above.

As can be seen in figure 9, for material F2 K₁ =0,0924 and for material F4, K₁ =0,4989. So F4 has a K₁ being too high.

For a good wetting of the first layer 2 it is important that the contact angle is less than 90 degrees with water, so that the moisture will spread easily over the first layer 2. The contact angle of nylon 6.6 is 68,3 degrees, of polyester 72,5 degrees and of polypropylene 102,01 degrees. Polypropylene as such will not be suitable but combined with other materials like polyester (PES) and/or the elastic portion of the fabric, good results can be obtained.

Furthermore the first layer 2 has a moisture regain of less than 5%, so that the first layer 2 does not have the possibility to keep a large amount of moisture inside the first layer 2. The moisture regain of polyamide (nylon) is 4,5%, of polyester 0,4% and of polypropylene 0%.

These three features determine the moisture behaviour of the first layer 2.

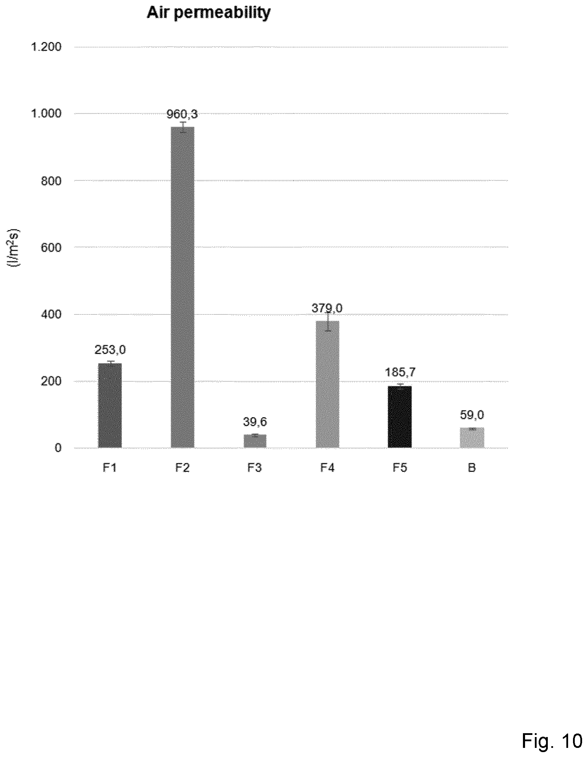

Figure 10 is a graph regarding air permeability of the materials as shown in figure 7. This has been measured according to NEN-EN-ISO 9237; 1995. Material F2 has an air permeability of 960,3 liter*metre⁻² *seconds⁻¹ and the material F4 has an air permeability of 379,0 liter*metre⁻² *seconds⁻¹. The first layer 2 has an air permeability of at least 50 liter*metre⁻² *seconds⁻¹, preferably of at least 375 liter*metre⁻² *seconds^{-1.}

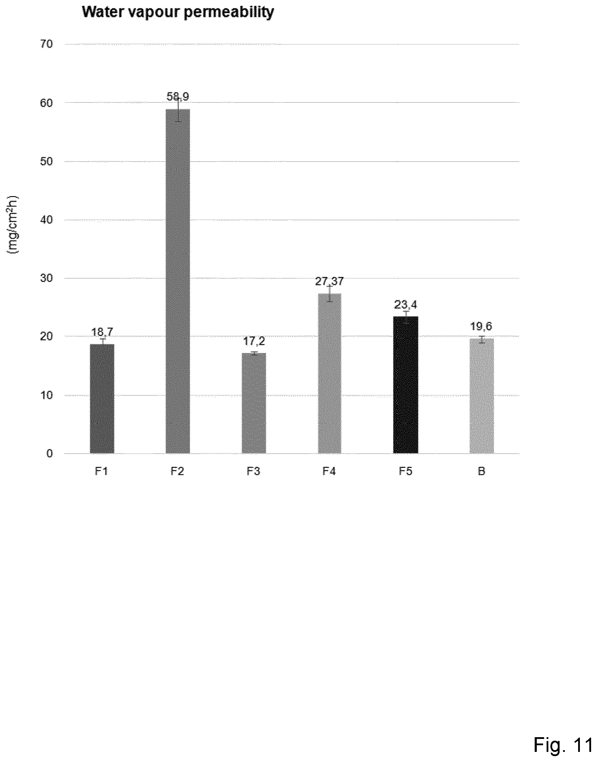

Figure 11 is a graph regarding water vapour permeability of the materials as shown in figure 7, which has been measured in a Mesdan Lab Vapour Tester 3395, adhering to EN ISO 14268.

Material F2 has a water vapour permeability of 58,9 mg*centimetre⁻²* hour⁻¹. Material F4 has a water vapour permeability of 27,37 mg*centimetre⁻²* hour⁻¹. The first layer has a water vapour permeability of at least 17 mg*centimetre⁻²* hour⁻¹. So all materials F1-F5, B are suitable. However, preferably the water vapour permeability is at least 25 mg*centimetre⁻²* hour⁻¹, and even more preferably of at least 55 mg*centimetre⁻²* hour⁻¹.

Figure 12 is a graph regarding thermal conductivity of the materials as shown in figure 7 as measured in an Atlas Fabric Touch Tester.

For each material F1-F5, B the thermal conductivity on the inner side and outer side of the first layer 2 is given.

Material F2 has a thermal conductivity of about 100 W·m⁻² · °C⁻¹. Material F4 has a thermal conductivity of about 99 W·m⁻² · °C⁻¹. The first layer should have a thermal conductivity of ≥ 95 W·m⁻² · °C⁻¹, preferably of ≥ 120 W·m⁻² · °C⁻¹.

The inner side and outer side in this graph are merely names to indicate different sides of the first layer 2. Preferably the side with the highest thermal conductivity is directed towards the skin. The average value of the values on both the inner side and outer side is the most relevant value.

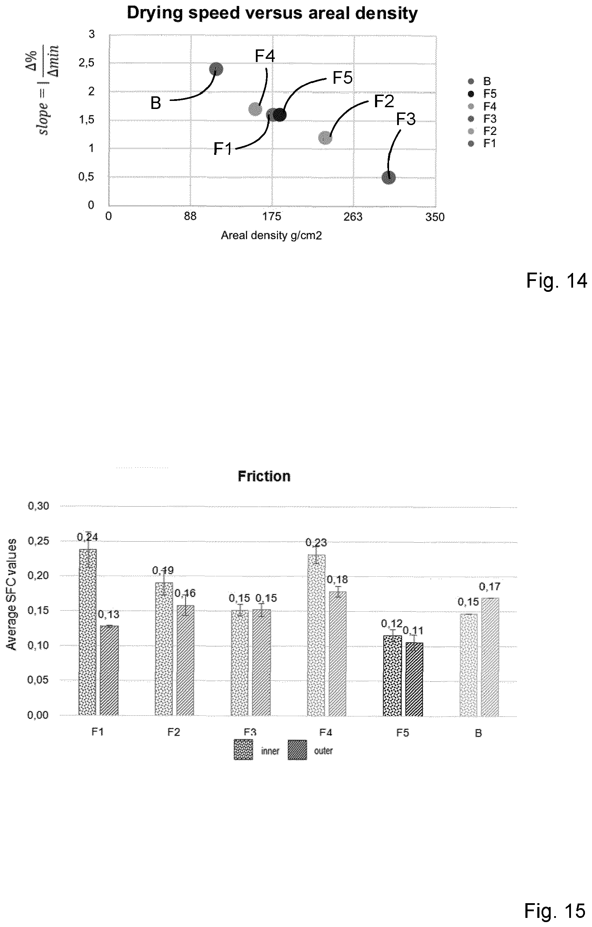

Figures 13 and 14 are graphs regarding drying speed of the materials as shown in figure 7.

Figure 13 shows the amount of water uptake in % versus time (minutes) for each material comparing to 65% humidity in figure 17 to reach its climatized weight from its wet weight as is defined at the total wet weight after 3 minutes immersion in water.

The first layer has a drying speed of ≥ 1 delta%/minute, preferably ≥ 1,5 delta%/minute. The value of the drying speed in delta%/minute is identical to the slope of the graph of figure 13.

Material F2 has a drying speed of 1,279 delta%/minute. Material F4 has a drying speed of 1,598 delta%/minute.

Figure 14 shows the relationship between the drying speed and areal density. The areal density of the materials F1-F5, B are given in figure 7.

Figure 15 is a graph regarding friction of the materials as shown in figure 7.

For each material F1-F5, B the average dynamic surface friction coefficient (SFC) on the inner side and outer side of the first layer 2 is given. Preferably the side with the lowest average dynamic surface friction coefficient (SFC) is directed towards the second layer.

Material F2 has an average SFC between 0,16 and 0,19. Material F4 has an average SFC between 0,18 and 0,23. Preferably the average SFC is as low as possible. The first layer has an average surface friction coefficient on a side directed towards the portion of the skin of less than 0,2.

Relevant for the friction is also the smoothness. Figure 16 is a graph regarding smoothness of the materials as shown in figure 7. Preferably the value regarding smoothness is as low as possible.

Material F2 has a smoothness of 0,64. Material F4 has a smoothness of 0,59.

The first layer has preferably a smoothness of less than 1, and more preferably less than 0,7.

Both friction and smoothness can be determined by a well known machine called Atlas fabric touch tester.

Figure 17 is a graph regarding moisture regain of the materials as shown in figure 7. Moisture absorbance capacity as mentioned in figure 17 is the same as moisture regain.

A climate chamber from a Mesdan lab instruments was used to determine the moisture holding capacity of the materials F1-F5, B in different humidity conditions. Firstly, samples were placed into the oven at 105 °C for 24 hours prior to the measurement of the bone-dry weight. Meanwhile, the amount of humidity in the climate chamber was set to the minimum level (10% relative humidity). However, the maximum moisture level decreased was up to 12,13% only in the machine. Once the humidity stabilized as 12,13%, the samples were placed into the climate for 24 hours and the weight of the samples was measured after 24 hours. Afterwards, the level of humidity was increased to 65% to calculate the weight of samples in climatized conditions. Lastly, the humidity in the climate chamber was increased to 92,7% which is the maximum humidity level in this machine. After waiting for 24 hours, the weight of the samples was measured again. All tests repeated three times. The temperature was set at 20,3°C during all the experiments in the climate chamber.

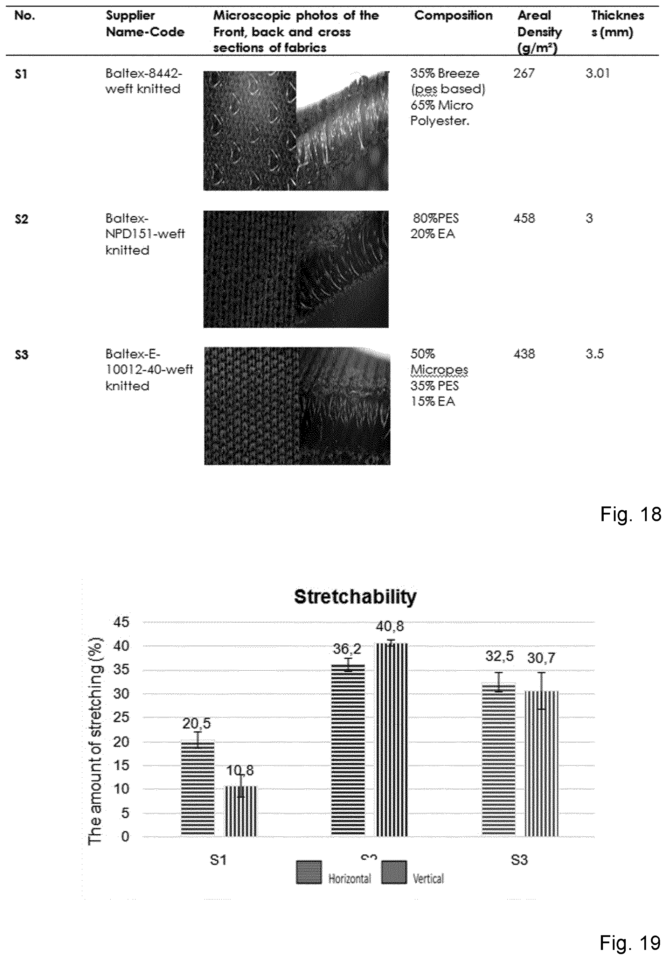

Lower moisture absorbing capacity enables fast transportation of moisture and heat as less sweat is going to be trapped. Figure 18 shows that the lowest moisture capacity is of F2 followed by F4 and the benchmark B. All these three fabrics are mainly made from polyester. On the other hand, the highest capacity is found for the fabrics of F3 and F1 which consist mostly of polyamide. Results show that fabrics with polyamide have a higher tendency to absorb the moisture than the fabrics with polyester as polyamide has higher moisture regain than polyester.

The first layer has a moisture regain of preferable less than 1,5% weight increase.

Figure 18 is an overview of several materials S1-S3 of the second layer 5 of the device 1 according to the invention.

The materials S1-S3 are mainly polyester (PES) based with elastane. These are products from Baltex (GB).

Figure 19 is a graph regarding stretchability of the materials S1-S3 as shown in figure 18. The stretchability is given for two directions, horizontal and vertical, extending perpendicular to each other on the surface of the second layer 2. Preferably the difference between the two direction is less than 25% to obtain a similar stretchability in both directions. Preferably a force between 7 and 10 N * cm⁻² is needed for an elongation of 50% Although S1 and S3 are suitable as well, S2 is the preferred second layer 5

The first layer 2 has a stretchability in the same order or similar to the stretchability of the second layer 5.

In case that the device of the present invention is being used to prevent the likelihood of ulcer formation on a portion of skin the stretchability of the first and second layer and the difference between the stretchability of the first and second layer are relevant. In case that the device of the present invention is being used to prevent night sweats the stretchability is less important.

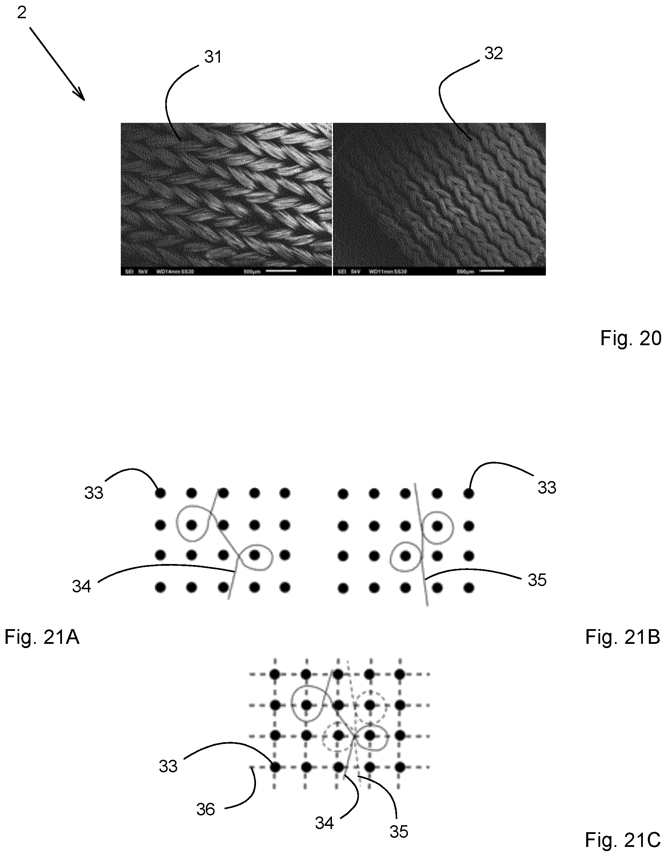

Figure 20 is a photo showing the inner side 31 (usually called the front side of the fabric) and outer side 32 (usually called the back side of the fabric) of the first layer 2 comprising a warp knitted two way structure called "Locknit" in the US and "Charmeuse" in Europe. F2 has such a structure.

Such two way structure is being made in a manner as shown in figures 21A and 21B. Figure 21A shows loopings of yarn 34 at subsequent relative positions 33 within an apparatus to produce the inner side 31 and figure 21B shows loopings of yarn 35 at subsequent relative positions 33 within the apparatus to produce the outer side 32.

In figure 21C the positions 33, yarns 34 and 35 on the inner side 31 and outer side 32 as well as a mesh 36 located between the inner side 31 and outer side 32 are shown.

During a test a first layer of F2 having a warp knitted two way structure was positioned on second layer comprising the spacer fabric. The spacer fabric was AMES DISTO, article number 340-40-135. This is a 100% polyester spacer fabric, with a weight of 520 gr/m2 and an average thickness of 4 millimetre. The mesh densities are 17 courses per centimetre and 9,5 wales/centimetre. The compression strength is 4 kPa. The two fabrics were positioned on top of each other. In order to ensure a good contact area, they were gently pressed on top of each other for 15 seconds. Subsequently a droplet of coloured liquid was placed on F2. With a chronometer the time was measured in which full immersion of the droplet in the two fabrics was achieved (clearly visible as the glowing droplet surface disappeared.

When F2 was positioned with the so-called front side up on the front side of the spacer fabric the average droplet disappearance/immersion time was 8,4 seconds. The backside was positioned against the front side of the spacer fabric.

When F2 was positioned with the so-called back side up on the front side of the spacer fabric the average droplet disappearance/immersion time was 5,9 seconds. The front side was positioned against the front side of the spacer fabric.

The results of the test show the great advantage of using the warp knitted two-way fabric with the Locknit/charmeuse structure with the backside on the skin and the front side on the interchange with the spacer fabric.

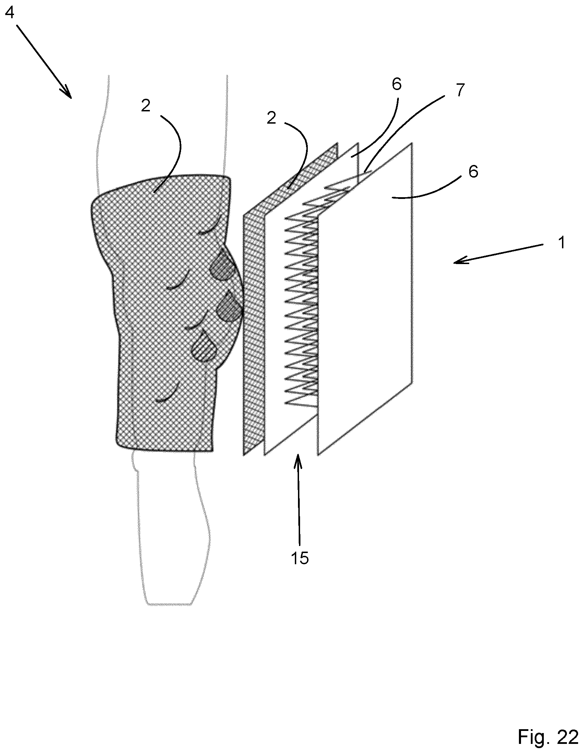

Figure 22 is a side view of another embodiment of an article according to the invention comprising the device 1 as shown in figure 1, as worn by a user 4, wherein the user 4 also wears a clothing made of the same material as the first layer.

It is also possible to make the first and second layer 2, 5 from the same material, like for example from material F4 comprising 94-96 % polyester and 4-6 % elastic fiber.

### LIST OF REFERENCE SIGNS

- 1: device
- 2: first layer
- 3: skin
- 4: user
- 5: second layer
- 6: channel
- 7: moisture
- 11: device
- 12: first layer
- 15: second layer
- 21: pair of trousers
- 22: spot
- 23: sock
- 24: pillow case
- 25: sheet
- 26: pillow
- 27: seam
- 28: opening
- 31: inner side
- 32: outer side
- 33: position
- 34: yarn
- 35: yarn
- 36: mesh
- B: material
- H: height
- F1: material
- F2: material
- F3: material
- F4: material
- F5: material
- P1: arrow
- P2: arrow
- P3: arrow
- P4: arrow
- P5: arrow
- S1: material
- S2: material
- S3: material
- T: time

## Claims

1. A device (1, 11) for moisture regulation on a portion of skin (3) of a living being, which device (1, 11) is at least to be located between the portion of skin (3) of the living being and an object proximate that portion of the skin (3) of the living being, the device comprising at least a first layer (2, 12) comprising of a tricot fabric and directed in use with an outer side towards the portion of the skin (3) and at least a second layer (5, 15) being in contact with an inner side of the first layer (2, 12) at a side remote of the portion of the skin (3), **characterized in that**:
- the first layer (2, 12) directed in use towards the portion of the skin (3)
• has a moisture regain of less than 5 percent
• has wicking according to DIN53924, with a relation between a vertical rising height H (cm) and the square root of a rising time T (sec) being H = K₁*T^{0,5} wherein 0,09 ≤ K₁ ≤ 0,2
- the second layer (5, 15) being in contact with the first layer (2, 12) at a side remote of the portion of the skin (3)
• comprises a spacer fabric being porous in a direction perpendicular as well as parallel to the surface of the second layer,
• has a compression resistance of greater than or equal to 4 kPa, more preferably greater than or equal to 10.5 kPa at 40 percent compression.
• has wicking according to DIN53924, with a relation between a vertical rising height H (cm) and the square root of a rising time T (sec) being H = K₂*T^{0,5} wherein K₂ is greater than K₁,

2. A device (1, 11) according to claim 1, **characterized in that** the inner side of the first layer (2, 12) is smoother and/or denser than the outer side of the first layer (2, 12).

3. A device (1, 11) according to claim 1 or 2, **characterized in that** the tricot of the first layer (2, 12) has a warp-knitted structure.,

4. A device (1, 11) according to claim 2 or 3, **characterized in that** between the inner side and outer side of the first layer (2, 12) an elastic mesh is located.

5. A device (1, 11) according to claim 4, **characterized in that** the mesh is made of a thermoplastic co-polyester elastomer (TPE-C).

6. A device (1, 11) according to claim 1 or 2, **characterized in that** the tricot of the first layer (2, 12) is made of a mixture of polyethylene terephthalate (PET) fibers and polybutylene terephthalate (PBT) fibers.

7. A device (1, 11) according to one of the preceding claims, **characterized in that** yarns of the tricot of the first layer (2, 12) are less than 120 denier.,

8. A device (1, 11) according to one of the preceding claims, **characterized in that** the first layer (2, 12) and/or the second layer (5, 15) comprises a synthetic polymer knitting of nylon, polyester, polypropylene, polyethylene, or a combination thereof with an elastic fiber like spandex, elastane, lycra,, a thermoplastic elastomer.

9. A device (1, 11) according to one of the preceding claims, **characterized in that** the first layer (2, 12) and/or the second layer (5, 15) comprises a content of elastic fiber between 2 - 30 percent.

10. A device (1, 11) according to one of the preceding claims, **characterized in that** the first layer and/or the second layer comprises 82-100 percent polyester and 0-18 percent elastic fiber.

11. A device (1, 11) according to one of the preceding claims, **characterized in that** the first layer and/or the second layer comprises a 30-70 percent polyethylene, 30-70 percent and polyester and 0-20 percent elastic fiber.

12. A device (1, 11) according to one of the preceding claims, **characterized in that** the second layer has a stretchability similar to the stretchability of the first layer.

13. A device (1, 11) according to one of the preceding claims, **characterized in that** the second layer has a thickness between 2,5 and 4 millimetre.

14. An article comprising a device (1, 11) according to above claims, like
- articles of clothing, - articles of covering cushioning devices like sheets, mattresses and pillows,
- articles of covering the skin touching surfaces of various medical devices, like orthotics, braces.

15. A set comprising a device according to one of the preceding claims as well as a clothing of a similar material as the first layer.

## Patentansprüche

1. Vorrichtung (1, 11) zur Feuchtigkeitsregulierung auf einem Teil der Haut (3) eines Lebewesens, wobei die Vorrichtung (1, 11) zumindest zwischen dem Teil der Haut (3) des Lebewesens und einem Objekt in der Nähe des Teils der Haut (3) des Lebewesens angeordnet werden soll, wobei die Vorrichtung zumindest eine erste Lage (2, 12), die aus einem Trikotstoff besteht und bei der Verwendung mit einer Außenseite auf den Teil der Haut (3) gerichtet ist, und mindestens eine zweite Lage (5, 15), die mit einer Innenseite der ersten Lage (2, 12) an einer von dem Teil der Haut (3) entfernten Seite in Kontakt steht, umfasst, **dadurch gekennzeichnet, dass**:
- die erste Lage (2, 12), die bei der Verwendung auf den Teil der Haut (3) gerichtet ist,
• eine Feuchtigkeitsaufnahme von weniger als 5 Prozent aufweist
• eine Dochtwirkung gemäß DIN 53924 aufweist, wobei die Beziehung zwischen einer vertikalen Steighöhe H (cm) und der Quadratwurzel einer Steigzeit T (s) wie folgt lautet: H = K₁*T^{0,5}, wobei 0,09≤K₁≤0,2
- die zweite Lage (5, 15), die an einer von dem Teil der Haut (3) abgewandten Seite mit der ersten Lage (2, 12) in Kontakt steht,
• ein Abstandsgewirke umfasst, das sowohl in einer Richtung senkrecht als auch parallel zur Oberfläche der zweiten Lage porös ist,
• eine Kompressionsfestigkeit von größer oder gleich 4 kPa, besonders bevorzugt größer oder gleich 10,5 kPa, bei 40 Prozent Kompression aufweist,
• eine Dochtwirkung gemäß DIN 53924 aufweist, wobei die Beziehung zwischen einer vertikalen Steighöhe H (cm) und der Quadratwurzel einer Steigzeit T (s) wie folgt lautet: H = K₂*T^{0,5}, wobei K₂ größer als K₁ ist.

2. Vorrichtung (1, 11) nach Anspruch 1, **dadurch gekennzeichnet, dass** die Innenseite der ersten Lage (2, 12) glatter und/oder dichter als die Außenseite der ersten Lage (2, 12) ist.

3. Vorrichtung (1, 11) nach Anspruch 1 oder 2, **dadurch gekennzeichnet, dass** das Trikot der ersten Lage (2, 12) eine Kettenwirkstruktur hat.

4. Vorrichtung (1, 11) nach Anspruch 2 oder 3, **dadurch gekennzeichnet, dass** sich zwischen der Innenseite und der Außenseite der ersten Lage (2, 12) ein elastisches Netz befindet.

5. Vorrichtung (1, 11) nach Anspruch 4, **dadurch gekennzeichnet, dass** das Netz aus einem thermoplastischen Co-Polyester-Elastomer (TPE-C) besteht.

6. Vorrichtung (1, 11) nach Anspruch 1 oder 2, **dadurch gekennzeichnet, dass** das Trikot der ersten Lage (2, 12) aus einer Mischung von Polyethylenterephthalat(PET)-Fasern und Polybutylenterephthalat(PBT)-Fasern besteht.

7. Vorrichtung (1, 11) nach einem der vorhergehenden Ansprüche, **dadurch gekennzeichnet, dass** Garne des Trikots der ersten Lage (2, 12) eine Feinheit von weniger als 120 Denier aufweisen.

8. Vorrichtung (1, 11) nach einem der vorhergehenden Ansprüche, **dadurch gekennzeichnet, dass** die erste Lage (2, 12) und/oder die zweite Lage (5, 15) ein synthetisches Polymergestrick aus Nylon, Polyester, Polypropylen, Polyethylen oder einer Kombination daraus mit einer elastischen Faser wie Spandex, Elastan, Lycra, einem thermoplastischen Elastomer umfasst.

9. Vorrichtung (1, 11) nach einem der vorhergehenden Ansprüche, **dadurch gekennzeichnet, dass** die erste Lage (2, 12) und/oder die zweite Lage (5, 15) 2 - 30 Prozent elastische Fasern umfasst.

10. Vorrichtung (1, 11) nach einem der vorhergehenden Ansprüche, **dadurch gekennzeichnet, dass** die erste Lage und/oder die zweite Lage 82-100 Prozent Polyester- und 0-18 Prozent elastische Fasern umfasst.

11. Vorrichtung (1, 11) nach einem der vorhergehenden Ansprüche, **dadurch gekennzeichnet, dass** die erste Lage und/oder die zweite Lage 30-70 Prozent Polyethylen-, 30-70 Prozent Polyester- und 0-20 Prozent elastische Fasern umfasst.

12. Vorrichtung (1, 11) nach einem der vorhergehenden Ansprüche, **dadurch gekennzeichnet, dass** die Dehnbarkeit der zweiten Lage ähnlich der Dehnbarkeit der ersten Lage ist.

13. Vorrichtung (1, 11) nach einem der vorhergehenden Ansprüche, **dadurch gekennzeichnet, dass** die zweite Lage eine Dicke zwischen 2,5 und 4 Millimeter aufweist.

14. Artikel, umfassend eine Vorrichtung (1, 11) nach einem der vorhergehenden Ansprüche, wie z. B.
- Kleidungsartikel, - Bezüge für Polster wie Laken, Matratzen und Kissen,
- Bezüge für hautberührende Oberflächen verschiedener medizinischer Geräte wie Orthesen und Schienen.

15. Set, umfassend eine Vorrichtung nach einem der vorhergehenden Ansprüche sowie Kleidung aus einem ähnlichen Material wie die erste Lage.

## Revendications

1. Dispositif (1, 11) destiné à réguler l'humidité sur une partie de la peau (3) d'un être vivant, lequel dispositif (1, 11) doit au moins être placé entre la partie de la peau (3) de l'être vivant et un objet à proximité de cette partie de la peau (3) de l'être vivant, le dispositif comprenant au moins une première couche (2, 12) constituée d'un tricot et orientée à l'usage avec un côté extérieur vers la partie de la peau (3) et au moins une deuxième couche (5, 15) en contact avec un côté intérieur de la première couche (2, 12) sur un côté à l'écart de la partie de la peau (3), **caractérisé en ce que** :
- la première couche (2, 12) orientée à l'usage vers la partie de la peau (3)
• a un taux de reprise d'humidité de moins de 5 pour cent,
• a un effet de mèche selon DIN53924, avec une relation entre une hauteur d'ascension verticale H (cm) et la racine carrée d'un temps d'ascension T (s) qui est H = K₁*T^{0,5} où 0, 09 ≤ K₁ ≤ 0,2 ;
- la deuxième couche (5, 15) en contact avec la première couche (2, 12) sur un côté à l'écart de la partie de la peau (3)
• comprend un tissu d'espacement qui est poreux dans une direction perpendiculaire ainsi que parallèle à la surface de la deuxième couche,
• a une résistance à la compression supérieure ou égale à 4 kPa, mieux encore supérieure ou égale à 10,5 kPa à 40 pour cent de compression,
• a un effet de mèche selon DIN53924, avec une relation entre une hauteur d'ascension verticale H (cm) et la racine carrée d'un temps d'ascension T (s) qui est H = K₂*T^{0,5} où K₂ est supérieur à K₁.

2. Dispositif (1, 11) selon la revendication 1, **caractérisé en ce que** le côté intérieur de la première couche (2, 12) est plus lisse et/ou plus dense que le côté extérieur de la première couche (2, 12).

3. Dispositif (1, 11) selon la revendication 1 ou 2, **caractérisé en ce que** le tricot de la première couche (2, 12) a une structure à mailles jetées.

4. Dispositif (1, 11) selon la revendication 2 ou 3, **caractérisé en ce qu'**entre le côté intérieur et le côté extérieur de la première couche (2, 12), on trouve un treillis élastique.

5. Dispositif (1, 11) selon la revendication 4, **caractérisé en ce que** le treillis est constitué d'un élastomère de copolyester thermoplastique (TPE-C).

6. Dispositif (1, 11) selon la revendication 1 ou 2, **caractérisé en ce que** le tricot de la première couche (2, 12) est constitué d'un mélange de fibres de téréphtalate de polyéthylène (PET) et de fibres de téréphtalate de polybutylène (PBT).

7. Dispositif (1, 11) selon une des revendications précédentes, **caractérisé en ce que** les fils du tricot de la première couche (2, 12) font moins de 120 deniers.

8. Dispositif (1, 11) selon une des revendications précédentes, **caractérisé en ce que** la première couche (2, 12) et/ou la deuxième couche (5, 15) comprennent un tricot en polymère synthétique de nylon, polyester, polypropylène, polyéthylène ou une combinaison de ceux-ci avec une fibre élastique comme le spandex, l'élasthane, le lycra, un élastomère thermoplastique.

9. Dispositif (1, 11) selon une des revendications précédentes, **caractérisé en ce que** la première couche (2, 12) et/ou la deuxième couche (5, 15) comprennent une teneur en fibre élastique comprise entre 2 et 30 pour cent.

10. Dispositif (1, 11) selon une des revendications précédentes, **caractérisé en ce que** la première couche et/ou la deuxième couche comprennent 82-100 pour cent de polyester et 0-18 pour cent de fibre élastique.

11. Dispositif (1, 11) selon une des revendications précédentes, **caractérisé en ce que** la première couche et/ou la deuxième couche comprennent 30-70 pour cent de polyéthylène, 30-70 pour cent de polyester et 0-20 pour cent de fibre élastique.

12. Dispositif (1, 11) selon une des revendications précédentes, **caractérisé en ce que** la deuxième couche a une extensibilité similaire à l'extensibilité de la première couche.

13. Dispositif (1, 11) selon une des revendications précédentes, **caractérisé en ce que** la deuxième couche a une épaisseur comprise entre 2,5 et 4 millimètres.

14. Article comprenant un dispositif (1, 11) selon les revendications précédentes, comme
- des articles vestimentaires, des articles de revêtement de dispositifs rembourrés comme des draps, des matelas et des oreillers,
- des articles de revêtement des surfaces en contact avec la peau de divers dispositifs médicaux, comme des dispositifs orthopédiques, des attelles.

15. Ensemble comprenant un dispositif selon une des revendications précédentes ainsi qu'un vêtement dans un matériau similaire à celui de la première couche.
